# EUROPEAN PATENT SPECIFICATION

(11) **EP 2 802 331 B1**
(45) Date of publication and mention of the grant of the patent: **12.12.2018**
(21) Application number: 13700826.4
(22) Date of filing: 10.01.2013
(51) Int. Cl.: A61K 31/5575, A61P 27/02

(54) **TOPICAL TREATMENT FOR CHEMOTHERAPY INDUCED EYELASH LOSS OR HYPOTRICHOSIS USING PROSTAMIDE F2 ALPHA AGONISTS**
TOPISCHE BEHANDLUNG EINES CHEMOTHERAPIEINDUZIERTEN AUGENWIMPERNVERLUSTS ODER EINER HYPOTRICHOSE MIT PROSTAMID-F2-ALPHA-AGONISTEN
TRAITEMENT TOPIQUE DE LA PERTE DE CILS OU DE L'HYPOTRICHOSE INDUITE PAR LA CHIMIOTHÉRAPIE À L'AIDE D'AGONISTES ALPHA F2 DE TYPE PROSTAMIDE

(30) Priority: 10.01.2012 US 201261584877 P; 16.03.2012 US 201261611920 P
(43) Date of publication of application: 19.11.2014
(73) Proprietor: ALLERGAN, INC., Irvine, CA 92612 (US)
(72) Inventor: AHLUWALIA, Gurpreet, Tustin, California 92705 (US); WHITCUP, Scott M., Laguna Hills, California 92653 (US); BEDDINGFIELD, Frederick C., Pacific Palisades, California 90272 (US); EDWARDS, Sydney G., Aliso Viejo, California 92656 (US)
(74) Representative: Hoffmann Eitle
(86) International application number: PCT/US2013/021023
(87) International publication number: WO 2013/106565

(56) References cited:
- WO-A1-2011/119748
- US-A1- 2007 160 562
- US-A1- 2011 002 866
- MORRIS CARRIE L ET AL: "The role of bimatoprost eyelash gel in chemotherapy-induced madarosis: an analysis of efficacy and safety", INTERNATIONAL JOURNAL OF TRICHOLOGY, MEDKNOW PUBL, INDIA, vol. 3, no. 2, 1 July 2011 (2011-07-01), pages 84-91, XP009166986, ISSN: 0974-9241, DOI: 10.4103/0974-7753.90809
- FAGIEN STEVEN: "Management of hypotrichosis of the eyelashes: Focus on bimatoprost.", CLINICAL, COSMETIC AND INVESTIGATIONAL DERMATOLOGY 2010, vol. 3, 2010, pages 39-48, XP009166922, ISSN: 1178-7015
- LAW S K: "Bimatoprost in the treatment of eyelash hypotrichosis", CLINICAL OPHTHALMOLOGY 2010 DOVE MEDICAL PRESS NZL, vol. 4, no. 1, 2010, pages 349-358, XP009166954, ISSN: 1177-5467

## Description

### FIELD OF THE INVENTION

The present invention is directed to bimatoprost for use in methods of treatment of post-chemotherapeutic hypotrichosis. More specifically, the present invention is directed to compositions comprising bimatoprost for use in methods of treatment of post-chemotherapeutic hypotrichosis.

### BACKGROUND OF THE INVENTION

Eyelashes, in addition to their contribution to appearance, serve a functional role by protecting sensitive eye structures against foreign particles entering the eye. The nerve plexus that surrounds hair follicles has a very low threshold for excitation (Moses, 1970); as a result, dust or other particles that may come into contact with the eyelash hair fiber are sufficient stimuli to produce a blink reflex, thereby protecting the eye. In terms of the aesthetic function of eyelashes, eyelash prominence has been observed to be related to the attractiveness of individuals, with long, thick eyelashes considered to be a desirable physical attribute with a positive psychological effect (Shaikh and Bodla, 2006).

Inadequate or not having enough eyelashes is known as hypotrichosis of the eyelashes. Etiologies of hypotrichosis of the eyelashes in an adult population include idiopathic hypotrichosis, alopecia-inducing medication (e.g., chemotherapeutic agents) and underlying cutaneous or systemic diseases/conditions (eg, alopecia areata or hypothyroidism).

In healthy adults, eyelash hypotrichosis is often idiopathic and may be related to age. There is an inverse relationship between age and length of eyelashes; younger populations naturally tend to have longer eyelashes, while older populations tend to have shorter eyelashes (Pucci, 2005). For this reason, many otherwise healthy adults experience hypotrichosis as a consequence of aging.

A treatment is available for the natural hypotrichosis condition which may be result of person's genetic makeup or could be age related. Bimatoprost solution 0.03% (LATISSE®) is marketed for the treatment of hypotrichosis of the eyelashes. Bimatoprost is a synthetic prostamide. Topical application of bimatoprost solution can be used in normal healthy adults with inadequate amount of eyelashes or subject who want to further enhance the prominence of their eyelashes (Yoelin, 2010). Treatment with bimatoprost has been demonstrated to increase the percentage of eyelash follicles in anagen, which accounts for its ability to lengthen eyelashes. Bimatoprost-induced stimulation of melanogenesis in melanocytes present in dermal papilla which are responsible for hair shaft pigmentation results in darker eyelashes and, at the same time, appears to increase the size of the dermal papilla and hair bulb, affecting lash thickness and fullness (Cohen, 2010; Fagien, 2010; Law, 2010).

In contrast to the natural eyelash hypotrichosis condition where the hair follicle is normal except it produces shorter and inadequate amount of eyelashes, chemotherapy treatment results in damage to the hair follicle components that make the hair fiber such that after the chemotherapy drug treatment, the natural eyelashes either fall off completely or result in patchy hair loss. Chemotherapeutic agents are well known for their ability to cause hair loss. Other drugs that can cause hair loss to varied degrees include anticoagulants, antithyroid drugs, oral contraceptives, lithium, interferons, antihyperlipidemic drugs, and retinoids (Tosti et al, 1994). Chemotherapy-induced hair loss is known to result from the direct toxic insult to rapidly dividing cells of the hair follicle (Trueb, 2009). During the anagen phase of the hair cycle, the epithelial compartment of the follicle undergoes proliferation, with the greatest proliferative activity occurring in the bulb matrix cells as they build up the hair shaft. When cell mitosis abruptly ceases as a result of cytotoxic therapy, the partially keratinized hair shaft weakens and falls out, resulting in anagen dystrophic effluvium (Ulrich et al, 2008). In addition, some chemotherapeutic agents can cause apoptosis (ie, programmed cell death) in the follicular epithelium resulting in premature transitioning from anagen to catagen phases of the hair cycle; this process is known as telogen effluvium (Ulrich et al, 2008). The consequence of these processes is hair shedding, which can begin within 1 to 3 weeks and is often complete within 1 to 2 months after beginning chemotherapy (Trueb, 2009). Hair loss occurs with an estimated incidence of 65% in adult patients receiving chemotherapy (Trueb, 2009). While eyelash loss can be part of the experience of chemotherapy-induced hair loss (Trueb, 2009), there are no reliable data in the published literature that specifically address the incidence of eyelash loss due to chemotherapy. However, the known mechanism by which chemotherapy induces alopecia indicates that any active hair follicle in anagen would be susceptible, including scalp, body, eyebrow, and eyelash hair.

For most cancer treatments, after the chemotherapy regimen is completed, the patient recovers from the treatment side effects relatively quickly, ie, most side effects of chemotherapy resolve within a few weeks of the last treatment; however, hair growth can continue to be depressed for a period of time. It can take several months to a year, or even longer in some subjects, for hair growth to restore to pre-chemotherapy levels. Moreover, when the hair does recover early, it is generally much finer and thinner than the original hair and can take several hair cycles to restore to the pre-chemotherapy levels.

Hair loss is known to be one of the most psychologically upsetting side effects of cancer therapy (Botchkarev, 2003, Lemieux et al, 2008; Hunt, 2005); it has been described by patients as a constant reminder of their illness and is associated with a loss of control, an altered sense of self, and reduced social functioning (Beisecker et al, 1997; Cowley, 2000; Freedman, 1994; Luoma and Hakamies-Blomqvist, 2004; Richer and Ezer, 2002; Williams et al, 1999). The loss not just of scalp hair but body hair can lead to psychosocial problems such as diminished quality of life expressed as anxiety, depression, and low self-esteem (Ulrich et al, 2008).

In the focus group studies, patients stated that the loss of eyelashes and eyebrows was worse than the loss of scalp hair because the latter could be easily concealed by a wig, whereas there was no way to make their eyelashes look "normal". False eyelashes were not a reasonable treatment in the opinion of the respondents because they did not have enough natural eyelashes to help the glue adhere to their eyelid margins. Moreover, such measures can result in severe irritation and skin damage and are therefore not ideal, especially in the post-chemotherapy population. In focus-group studies, many postchemotherapy patients commented that their eyelashes never fully recovered to their pre-chemotherapy levels. Even though they noticed some re-growth, most complained that their eyelashes were sparse (ie, gaps between lashes), short, and lighter in color.

Currently there are no treatments available for chemotherapy induced eyelash loss. We discovered that treatment with LATISSE (bimatoprost 0.03% solution) restores eyelash growth and prominence quickly compared with the natural course of slower recovery. Thus the protective function of eyelashes is resumed earlier in treated patients as compared to non-treated patients. The postchemotherapy patients treated with bimatoprost 0.03% solution express a higher overall satisfaction with their eyelashes as compared to patients treated with vehicle. Bimatoprost treatment in postchemotherapy patients also restored length, thickness/fullness and darkness of eyelashes.

In Int. J Trichology, Jul-Dec 2011, 3(2), 84-91, a randomized, single-blinded, prospective, internally controlled trial was reported which compared bimatoprost eyelash gel in relation to eyelash enhancement of madarosis patients. Forty eyelids of 20 chemotherapy-treated breast cancer patients were randomized to treatment or control (fellow eyelid). Both patient and surgeon (blindly) evaluated bimatoprost gel's effectiveness in improving eyelash appearance at baseline and at monthly intervals. It was concluded that Bimatoprost eyelash gel appears promising for chemotherapy-induced madarosis, and that patients may find the effects restorative and cosmetically enhancing.

US2007160562 relates to cosmeceuticals capable of stimulating the growth of hair, e.g., natural eyelashes, in a human subject. More specifically, this disclosure relates to delivery devices that provide for effective, targeted application of a therapeutically effective amount of the improved cosmeceuticals. According to the authors, the targeted application helps to limit and/or eliminate concerns about the cosmeceutical active ingredient effecting unintended areas of the body.

WO2011119748 relates to compositions including at least one prostaglandin or prostamide and methods for using these compositions for increasing hair growth on the scalp.

US2011002866 relates to the delivery of compositions comprising at least one prostaglandin analog to prevent or reduce hair loss (e.g. brittle hair growth, thin hair growth, short hair growth, sparse hair growth) or alopecia associated with chemotherapy.

### SUMMARY OF THE INVENTION

The present invention is directed to bimatoprost for the use in a method of growing eyelashes in post-chemotherapeutic patients as defined in claim 1. The present invention is also directed to bimatoprost for use in a method of preventing the loss of eyelashes during chemotherapeutic treatment as defined in claim 12. The present invention is also directed to bimatoprost for use in a method of preventing the loss of eyelashes prior to the start of chemotherapy. The present invention is also directed to bimatoprost for the use in a method of growing eyelashes or for use in a method of preventing the loss of eyelashes before, during and after chemotherapeutic treatment.

The present invention is applied as 0.03% w/v bimatoprost, which is available in the commercial product called LATISSE®. According to the present disclosure, the bimatoprost may be applied in concentrations 0.3% w/v to 0.001% w/v and including concentrations such as 1.0 % w/v, 0.9% w/v, 0.8% w/v, 0.7% w/v, 0.6% w/v, 0.5% w/v, 0.4% w/v, 0.3% w/v, 0.2% w/v, 0.1% w/v, 0.09% 0.08 % w/v, 0.07% w/v, 0.06% w/v, 0.05% w/v, 0.04% w/v, 0.03% w/v, 0.02% w/v, 0.01% w/v, 0.009% w/v, 0.008% w/v, 0.007% w/v, 0.006% w/v, 0.005% w/v, 0.004% w/v, 0.003% w/v, 0.002% w/v, 0.001% w/v, 0.009% w/v, 0.008% w/v, 0.007% w/v, 0.006% w/v, 0.005% w/v, 0.004% w/v, 0.003% w/v, 0.002% w/v, and 0.001% w/v bimatoprost.

Bimatoprost may be applied as a solution, emulsion, gel, foam, spray, ointment, cream, or other form suitable for administration to the eyelid margin. Bimatoprost may be in the form of a salt, pro-drug, analogs including esters of bimatoprost. The bimatoprost composition, including LATISSE ®, may also be applied in conjunction with other therapeutics known to grow hair such as Minoxidil® and Propecia®.

"Treatment", "treat" or "treating" can refer to curing any disease or condition or reducing or alleviating the symptoms of the disease or condition.

"Prevent", "preventing" or "prevention" can refer to stopping any disease, condition or symptoms or reducing symptoms in a clinically significant manner, particularly as compared to patients receiving no treatment at all.

Some embodiments of the present invention include the following paragraphs:
1) Bimatoprost for use in a method of growing eyelashes in patients undergoing chemotherapy, the method comprising applying 0.03% w/v bimatoprost to the eyelids before, during and after chemotherapeutic treatment, wherein the method is applied for at least 6 months after completing chemotherapeutic treatment.
2) The bimatoprost for use of paragraph 1 wherein the 0.03% bimatoprost is applied at least once a day.
3) The bimatoprost for use of paragraphs 1 and 2 wherein the method results in eyelashes which are longer, thicker and darker compared to patients receiving no treatment.
4) The bimatoprost for use of paragraph 1 wherein the method is applied for at least 12 months after completing chemotherapeutic treatment.
5) The bimatoprost for use of paragraph 3 wherein the number of eyelashes increases in comparison to post-chemotherapeutic patients who received no treatment.
6) The bimatoprost for use of paragraph 1 wherein the bimatoprost is added before, during and after post-chemotherapeutic treatment.
7) The bimatoprost for use of paragraph 2, wherein the method is applied twice a day.
8) The bimatoprost for use of paragraphs 2 and 7 wherein the bimatoprost is applied to the upper and lower eyelid margin of each eye.
9) The bimatoprost for use of paragraphs 1-8 wherein the method effectively treats post-chemotherapeutic hypotrichosis.
10) The bimatoprost for use of paragraph 1 wherein the eyelids include application to the upper and lower eyelid margin.
11) The bimatoprost for use of paragraph 1 further comprising the step of administering one selected from the group consisting of Minoxidil® and Propecia®.
12) The bimatoprost for use of claim 1 wherein the method results in lower incidence of conjunctival hyperemia, punctate keratitis, erythema of the eyelid, eye pruritis and skin hyperpigmentation than in patients receiving 0.03% w/v bimatoprost for treatment of idiopathic hypotrichosis.
13) Bimatoprost for use in a method of preventing loss of eyelashes in patients undergoing chemotherapy, the method comprising applying 0.03% w/v bimatoprost to the eyelids before, during, or after chemotherapeutic treatment, wherein the method is applied for at least 6 months after completing chemotherapeutic treatment.
14) The bimatoprost for use of paragraph 13 wherein the 0.03% bimatoprost is applied at least once a day.
15) The bimatoprost for use of paragraphs 13 and 14 wherein the method results in eyelashes which are longer, thicker and darker compared to patients receiving no treatment.
16) The bimatoprost for use of paragraph 13 wherein the method is applied for at least 12 months after completing chemotherapeutic treatment.
17) The bimatoprost for use of paragraph 12 wherein the number of eyelashes increases in comparison to post-chemotherapeutic patients who received no treatment.
18) The bimatoprost for use of paragraph 13 wherein the bimatoprost is added before, during and after post-chemotherapeutic treatment.
19) The bimatoprost for use of paragraph 13, wherein the method is applied twice a day.

### BRIEF DESCRIPTION OF THE DRAWINGS

Fig. 1 shows an Example of the Effect of Bim 0.03% on Eyelash Growth Compared to Vehicle - postchemotherapy population;
Fig. 2 Percentage of Subjects With at Least a 1-Grade Improvement in GEA Score - Postchemotherapy;
Fig. 3 Shows treatment Responders (%) Based on Primary Composite Variable by Month: Postchemotherapy (Intent-to-treat Population);
Fig. 4 Shows Mean Change From Baseline in Eyelash Length (mm) by Month: Postchemotherapy;
Fig. 5 is a plot of primary composite efficacy for the duration of the trial for subjects with idiopathic hyopotrichosis;
Fig. 6 is a plot of responder rates in bimatoprost-treated subjects by individual components of the primary composite efficacy measure for the duration of the for subjects with idiopathic hyopotrichosis;
Fig. 7 is a plot of improvement in eyelash length for the duration of the trial for subjects with idiopathic hyopotrichosis;
Fig. 8 is a plot of primary composite efficacy for the duration of the trial for subjects with chemotherapy-induced hyopotrichosis;
Fig. 9 is a plot of responder rates in bimatoprost-treated subjects by individual components of the primary composite efficacy measure for the duration of the trial for subjects with chemotherapy-induced hyopotrichosis; and,
Fig. 10 is a plot of improvement in eyelash length for the duration of the trial for subjects with chemotherapy-induced hyopotrichosis.

### DETAILED DESCRIPTION OF THE DRAWINGS

### Example 1

**Table I. List of Components and Quantitative Composition**

| Ingredients | Concentration (% w/v) | Concentration (mg/mL) | Function |
|---|---|---|---|
| Active ingredient | | | |
| Bimatoprost^{a} | 0.03 | 0.3 | Active ingredient |
| Other ingredients | | | |
| Benzalkonium chloride^{b} | 0.005 | 0.05 | Preservative |
| Sodium phosphate dibasicheptahydrate | 0.268 | 2.68 | Buffering agent |
| Citric acid monohydrate | 0.014 | 0.14 | Buffering agent |
| Sodium chloride | 0.83 | 8.3 | Tonicity agent |
| Hydrochloric acid^{c} and/or sodium hydroxide^{c} | Adjust to pH 7.2 | -7.4 | pH adjuster |
| Purified water | q.s. ad 100% | q.s. ad 1 mL | Vehicle |

### Clinical Data:

A clinical study was conducted that demonstrated the clinical benefits of bimatoprost 0.03% solution in treating eyelash loss resulting from chemotherapy treatment.

### Study Design and Structure:

This was a 1-year, multicenter, double-masked, randomized, parallel-group study to evaluate the safety and efficacy of bimatoprost solution 0.03% in increasing overall eyelash prominence following dermal application to the upper eyelid margins in normal adults and postchemotherapy adults exhibiting hypotrichosis of the eyelashes. Subjects enrolled in the study were adult subjects at least 18 years of age, with idiopathic or chemotherapy-induced hypotrichosis (Global Eyelash Assessment [GEA] score of 1 or 2) and had a score of 1 or 2 on each of the 3 items (16, 18, and 19) on the Eyelash Satisfaction Questionnaire (ESQ) Domain 2, which represented psychological impact of eyelash loss.

The full 12-month study consisted of 2 distinct 6-month treatment periods, treatment period 1 (TP1) and treatment period 2 (TP2). Eligible post-chemotherapy subjects were randomly assigned in a 3:1 ratio to receive bimatoprost or vehicle for TP1. In TP2, the subjects were either maintained on or switched to bimatoprost treatment.

A total of 130 subjects with chemotherapy-induced hypotrichosis were randomized. Of these, 96 subjects were randomized to the Bim 0.03% group and 34 subjects to the vehicle group. The overall mean age of the postchemotherapy subjects was 50.7 years (range 26 to 76 years), and the majority of the population was Caucasian (79.2%). All except 1 of the subjects enrolled were female (99.2%; 129/130). Per inclusion criteria, all enrolled subjects had a baseline GEA score of 1 (71.3%) or 2 (28.7%), with a similar distribution of GEA scores in both treatment groups at baseline. The mean total score ± SD of ESQ Domain 2 for was 3.9 ± 1.23. All enrolled subjects had a baseline ESQ score of 1 or 2 for items 16, 18, and 19 that relates to psychological impact of eyelash loss or hypotrichosis condition.

### Primary Composite Efficacy Endpoint:

The primary efficacy endpoint was the proportion of treatment responders at month 4 based on a composite endpoint, defined by: a) at least a 1-grade improvement from baseline in the GEA score, and b) at least a 3-point improvement from baseline in the total score for Domain 2 of the ESQ. The GEA is an investigator assessment of eyelash prominence and the ESQ score is patients own perception of their eyelashes.

### 6 month data:

After 4 months of daily treatment, in the post-chemotherapy subpopulation, the treatment responder rates based on the primary efficacy end point were 37.5% (36/96) in the bimatoprost 0.03% group and 18.2% (6/33) in the vehicle group. Data in the table below shows response rate by visit at month 1, 2, 4, and 6. A continuous improvement in efficacy is observed over the six month time period.

**Table II: Primary Composite Efficacy Variable: Treatment Responders by Visit**

| Visit | | Postchemotherapy population | | |
|---|---|---|---|---|
| | | Bim 0.03% (N=96) | Vehicle (N=34) | P-value^{b} |
| Month 1 | | 6/ 96 (6.3%) | 2/ 33 (6.1%) | >0.999^{c} |
| Month 2 | | 22/ 96 (22.9%) | 5/ 33 (15.2%) | 0.344 |
| Month 4 | | 36/ 96 (37.5%) | 6/ 33 (18.2%) | 0.041 |
| Month 6 | | 45/ 96 (46.9%) | 6/ 33 (18.2%) | 0.004 |

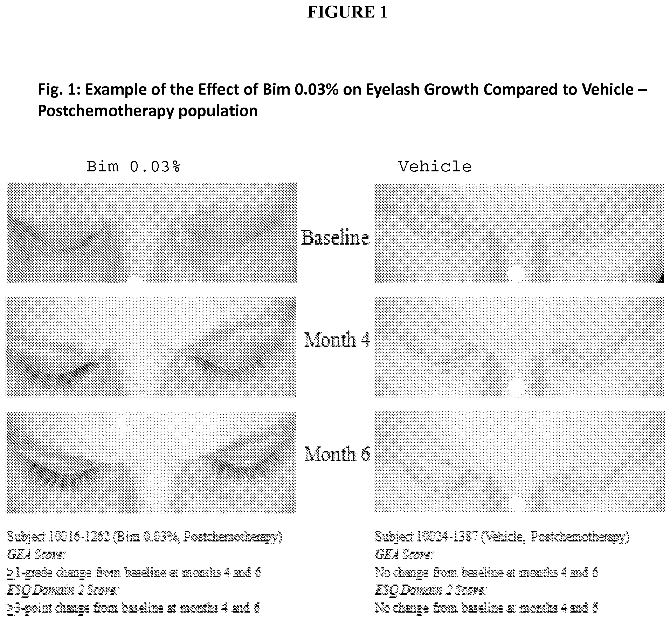

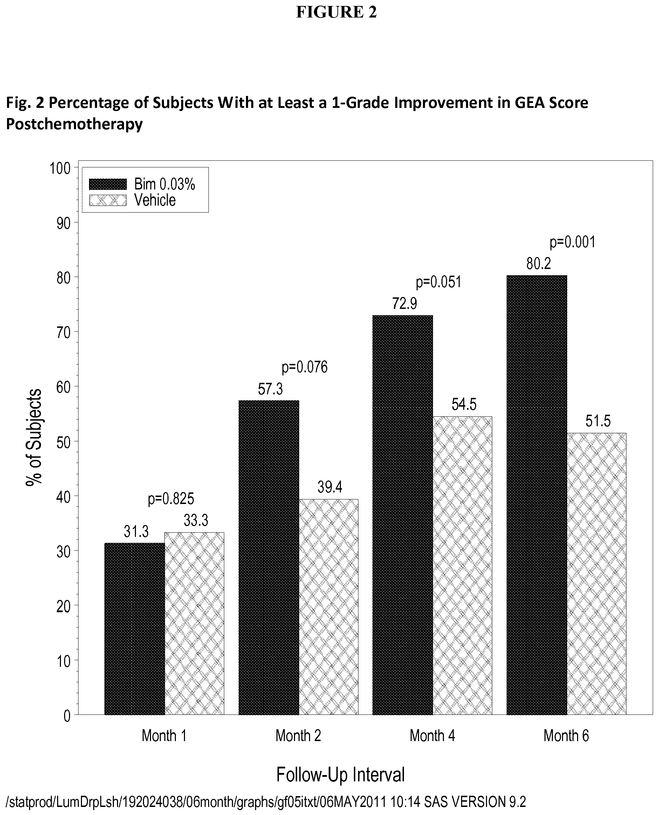

The response rate was also determined solely based on the investigator GEA scoring. As shown in Figure 1, the Bim 0.03% group had a higher responder rate at the month 2,4, and 6 visits compared with the vehicle group. The difference in responder rate, based on GEA of eyelash prominence, approached statistical significance at month 4 (p = 0.051) and was statistically significant at the month 6 visit (p = 0.001). The relatively high responder rate in the vehicle group of the post-chemotherapy population compared to the vehicle group of the normal adult population is attributable to the natural re-growth that occurs to some degree upon completion of chemotherapy treatment. Figure 2 shows the percentage of subjects with at least a 1-grade improvement in GEA Score - Post-chemotherapy.

Efficacy was also assessed using more conservative criteria of 2-grade improvement in GEA. At month 4, the responder rates for the 2-grade increase in the Bim 0.03% group was 36.5% (35/96) compared to vehicle response of 6.1% (2/33) for this 2 grade increase. In addition to the investigator global assessment (GEA) and subjects own assessment (ESQ), the eyelash length, thickness/fullness and darkness were measures using digital image analysis.

The mean change in eyelash length from baseline at month 4 was 1.48 mm in the Bim 0.03% group and 0.72 mm in the vehicle group. By month 6, the mean change in eyelash length from baseline was 1.99 mm for the Bim 0.03% group and 1.01 mm for the vehicle group.

The mean changes in eyelash thickness from baseline at month 4 were 0.67 mm² in the Bim 0.03% group and -0.05 mm² in the vehicle group. By month 6, the mean changes in eyelash thickness from baseline were 0.83 mm² for the Bim 0.03% group and 0.04 mm² for the vehicle group.

The mean change from baseline in eyelash darkness was greater in the Bim 0.03% than in the vehicle group. At the month 4 and 6 visits, it was -22.48 and -26.46, respectively, in the Bim 0.03% group and -11.25 and -10.19, respectively, in the vehicle group. The greater negative number on this measure reflects the greater intensity or darkness of eyelashes.

### Summary of efficacy data on effect of bimatoprost on increasing eyelash growth in post-chemotherapy population:

For the primary composite efficacy endpoint, the Bim 0.03% group had a statistically significantly higher responder rate than the vehicle group at month 4 (p = 0.041). At month 4, the responder rate was 37.5% (36/96) in the Bim 0.03% group and 18.2% (6/33) in the vehicle group. By month 6, the responder rate in the Bim 0.03% group increased to 46.9% (45/96), whereas there was no change in the vehicle group (18.2%, 6/33).

The Bim 0.03% group had a higher percentage of subjects with at least a 1-grade increase from baseline in GEA score compared to the vehicle group at all follow-up visits. The difference between the 2 groups approached statistical significance at month 4 (p = 0.051) and was statistically significantly different at the month 6 visit (p = 0.001). At the month 4 visit, 72.9% in the Bim 0.03% group and 54.5% in the vehicle group had at least a 1-grade increase from baseline in GEA score. By month 6, the percentage of responders increased to 80.2% in the Bim 0.03% group, whereas in the vehicle group it decreased to 51.5%.

The percentage of subjects with at least a 1-grade increase from baseline in GEA in the Bim 0.03% group of the postchemotherapy subpopulation (72.9%) was comparable to that of the normal adult subpopulation (74.3%) at month 4. Relative to the vehicle group in the normal adult subpopulation, the vehicle group in the postchemotherapy subpopulation showed higher GEA response at all visits which is likely related to some degree of natural regrowth in the postchemotherapy subpopulation

Statistically significant improvements from baseline in upper eyelash length, thickness, and darkness were seen in the Bim 0.03% group compared to the vehicle group at month 4 and month 6.

At month 4, 36.5% of subjects in the Bim 0.03% group of the postchemotherapy subpopulation had at least a 2-grade increase from baseline in GEA scores.

Statistically significant improvements in favor of Bim 0.03% group were observed for ESQ Domains 1 and 3 scores at months 4 and 6. For Domain 2, although the improvements were not statistically significantly different between the 2 treatment groups, the Bim 0.03% group had a higher mean change in total score from baseline than the vehicle group (2.8 versus 1.7).

### Postchemotherapy population (safety summary)

In the postchemotherapy subpopulation, 57.3% (55/96) of subjects in the Bim 0.03% group and 45.5% (15/33) of subjects in the vehicle group reported at least 1 adverse event over the first 6-month study period. Adverse events that were more common in the Bim 0.03% group (more than 5% of subjects) than in the vehicle group were conjunctival hyperaemia, punctate keratitis and eye pruritus. The majority of adverse events were reported as mild or moderate in severity. The treatment-related adverse events were reported by 27.1% (26/96) and 6.1% (2/33) of subjects in the Bim 0.03% and vehicle groups, respectively. Treatment-related adverse events reported by more than 1 subject in the Bim 0.03% group were conjunctival hyperaemia (12 subjects), punctate keratitis (7 subjects), eyelids pruritus (3 subjects), eye pruritus (3 subjects), skin hyperpigmentation (3 subjects) and eyelid irritation (2 subjects). The 2 treatment-related adverse events reported in the vehicle group were punctate keratitis (1 subject) and eyelids pruritus (1 subject).

None of the treatment-related adverse events were reported as severe, and none of them led to study or treatment discontinuation.

### 12-month data:

For subjects receiving bimatoprost for up to 12 months (Bim/Bim group), the efficacy demonstrated for the composite end point, ie, the proportion of responders increased from month 6 to the month 12 period as shown in the figure below. The responder rate, based on the primary efficacy composite measure, increased from 46.9% at month 6 to 61.5% at month 12. These data indicate continuous improvement seen in the postchemotherapy population through month 12 of treatment. These data also demonstrate that efficacy is maintained over 12 months of daily exposure, with no indication for development of any resistance to the treatment.

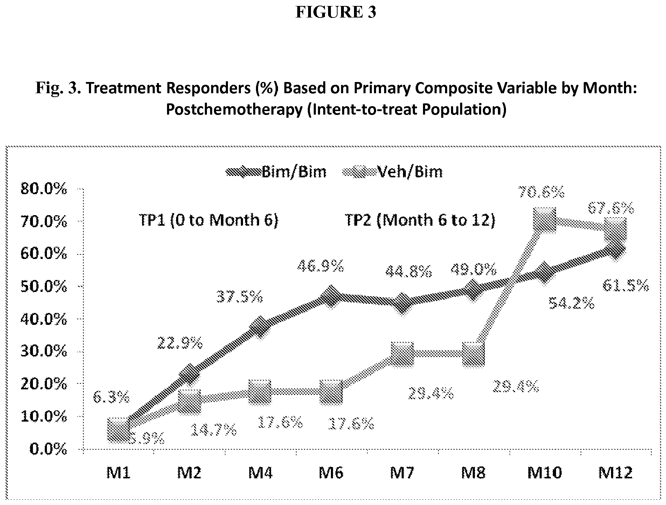

Subjects that received vehicle in the first 6 months of treatment and then switched to bimatoprost in TP2 (Veh/Bim groups), the drug effect was rapidly realized, the responder rate increased from 17.6% (6/34) at month 6 to 67.6% (23/34) at month 12 as shown in Figure 3.

### Eyelash Length

For the idiopathic hypotrichosis subpopulation treated for up to 12 months with bimatoprost, the mean eyelash length at baseline was 5.69 mm and increased by 1.44 mm at month 6 of treatment, and then remained fairly constant throughout the treatment period. This corresponds to a mean percent increase from baseline of 26.17% at month 6 and 25.86% at month 12, and a median percent increase from baseline of 22.4% at month 6 and 22.63% at month 12 This indicates that eyelash length increase is maintained, with no evidence of development of resistance, from month 6 through 12 of daily treatment.

### Mean Change ± Standard Deviation (SD) from Baseline in Eyelash Length (mm)

| | Postchemotherapy | |
|---|---|---|
| TP1/TP2/Vis it^{a} | Bim/Bim (N = 96) | Veh/Bim (N = 34) |
| Baseline | 4.86 ± 1.189 | 4.65 ± 1.413 |
| Month 4 | 1.48 ± 1.391 | 0.72 ± 1.396 |
| Month 6 | 1.99 ± 1.557 | 1.01 ± 1.275 |
| Month 10 | 2.14 ± 1.455 | 2.27 ± 1.439 |
| Month 12 | 2.01 ± 1.504 | 2.07 ± 1.442 |

| | | |
|---|---|---|
| Bim =bimatoprost 0.03%; TP1 = treatment period 1 (day 1 to month 6); TP2 = treatment period 2 (month 6 to 12); Veh = vehicle | | |

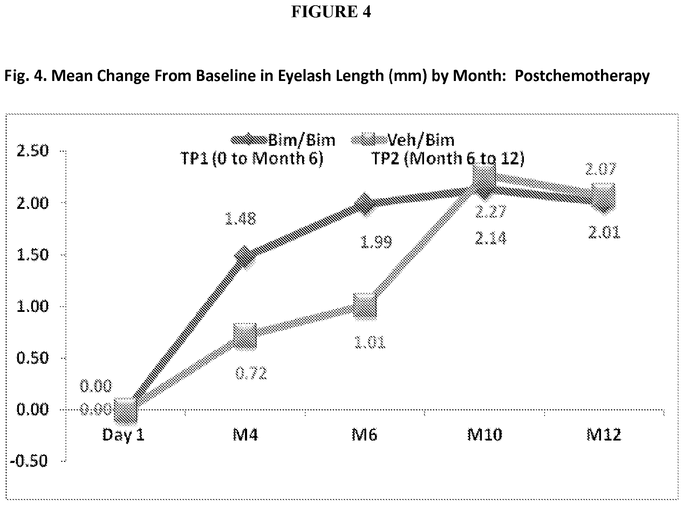

For the post-chemotherapy subjects treated for up to 12 months with bimatoprost, the mean eyelash length at baseline was 4.86 mm and increased by 1.99 mm at month 6 of treatment, and then remained fairly constant throughout the treatment period. This corresponds to a mean percent increase from baseline of 48.08% at month 6 and 49.88% at month 12, and a median percent increase from baseline of 37.84% at month 6 and 39.08% at month 12), again indicating that eyelash length increase is maintained, with no evidence of loss of effect upon continuous daily treatment from months 6 through 12 as shown in Fig. 4.

For the postchemotherapy subjects treated for up to 12 months with bimatoprost, the mean thickness at baseline was 0.39 mm², which increased by 0.83 mm² at month 6 of treatment, and then remained fairly constant throughout the treatment period. This corresponds to a mean percent increase from baseline of 428% at month 6 and 478% at month 12, and a median percent increase from baseline of 245% at month 6 and 212% at month 12, again the eyelash thickness increase was maintained, with no evidence of loss of effect upon continuous daily treatment from month 6 through 12.

### Mean Change ± Standard Deviation (SD) from Baseline in Average Progressive Eyelash Thickness (mm²)

| TP1/TP2/Visit^{a} | Po stchemotherapy | |
|---|---|---|
| | Bim/Bim (N = 96) | Veh/Bim (N = 34) |
| Baseline | 0.39 ± 0.302 | 0.67 ± 0.995 |
| Month 4 | 0.67 ± 0.514 | -0.05 ± 0.955 |
| Month 6 | 0.83 ± 0.576 | 0.04 ± 1.009 |
| Month 10 | 0.88 ± 0.516 | 0.63 ± 1.042 |
| Month 12 | 0.85 ± 0.575 | 0.58 ± 1.085 |

| | | |
|---|---|---|
| Bim = bimatoprost 0.03%; TP1 = treatment period 1 (day 1 to month 6); TP2 = treatment period 2 (month 6 to 12); Veh = vehicle | | |

### At Least a 2-grade Increase in Global Eyelash Assessment Score

A secondary analysis of the GEA component of the primary efficacy variable using a more stringent criterion was the percentage of subjects who experienced at least a 2-grade increase and a 3-grade increase from baseline on the GEA scale. For postchemotherapy hypotrichosis subjects treated for up to 12 months with bimatoprost, 45.8% of the subjects had at least a 2-grade increase in GEA at month 6, which increased to 57.3% at month 12. This indicates a progressive increase in eyelash prominence from month 6 to 12.

The postchemotherapy subjects treated with vehicle for the first 6 months and then switched to bimatoprost treatment (Veh/Bim) had only 8.8% (3/34) of the subjects with a 2-grade GEA increase at month 6; this increased to 50% by month 10 (4 months after starting bimatoprost treatment) and to 52.9% by month 12.

Efficacy in the post-chemotherapy hypotrichosis population showed a gradual increase in the number of responders through 12 months of treatment. Though an early peak in the percent responders was observed at month-6 (46.9%) and a minimal change between months-6 and -8, there was a gradual further increase to 54.2% at month-10 and an increase to 61.5% at month-12, indicating a continuous improvement in this population. A similar gradual increase in the percent responders was noted based on at least 1-grade increase in GEA or at least 3-point increase in ESQ Domain-2 from month-6 to the month-12 treatment. The GEA responders increased from 80.2 to 90.6% and the ESQ Domain-2 responders increased from 47.9 to 63.5% between month-6 and -12.

Majority of the common adverse events observed for the entire 12 month period were from the first 6 months of treatment, indicating that continuous treatment does not lead to increased incidence of adverse events. For example, the incidence rate for three of the most common AEs in the postchemotherapy population, conjunctival hyperaemia, punctate keratitis and eyelids pruritus was 15.6 %, 8.3% and 3.1%, respectively, in the first 6 months of treatment vs. only 1.1% (new AE) for each of these three events for months 6-12.

### Example 2

This is a long-term safety and efficacy study of bimatoprost ophthalmic solution 0.03% (LATISSE®) bimtoprost carried out in idiopathic and post-chemotherapy hypotrichosis populations. In this study, eyelash loss from chemotherapy was studied.

### Study Design:

A one-year, multicenter, randomized, double-masked, vehicle-controlled study. Adult post-chemotherapy and idiopathic eyelash hypotrichosis subjects were enrolled based on their score of 1 or 2 on a four point ordinal Global Eyelash Assessment (GEA) scale, and in addition having a low score on a PRO measure associated with 'psychological impact' of the condition, a domain-2 of the Eyelash Satisfaction Questionnaire (ESQ). The study involved two treatment periods of six months each. In the first treatment period, subjects for both populations were randomized 3:1 for QD bimatoprost: vehicle treatment. In the second 6-month treatment period, all subjects were moved to bimatoprost treatment, except for a group of bimatoprost treated idiopathic hypotrichosis subjects (n=55) who were switched to vehicle to investigate the effect of drug discontinuation. The study included 9 visits over the 12 month treatment period. The primary end point was the proportion of responders within each treatment group based on a composite measure of GEA and ESQ Domain-2 (investigator assessed eyelash prominence and subject's assessment of 'psychological impact' related to eyelashes) at month-4.

### Results:

A total of 368 subjects were randomized, 238 idiopathic and 130 post-chemotherapy. The primary efficacy end point was met for both idiopathic and post-chemotherapy populations. A baseline, majority of the post-chemotherapy subjects showed sparse, patchy eyelashes to near complete loss. In both populations, majority of the subjects (>70%) demonstrated increased eyelash prominence (≥1 grade GEA improvement) at month-4 following daily bimatoprost treatment. There were no drug related serious adverse events in the study.

In subjects with idiopathic hypotrichosis, 40.2% efficacy (a greater than 1-grade increase in GEA score and at least 3 point improvement in ESA domain score) was achieved at month 4, while only 6.8% of the vehicle treated subjects had a similar increase in GEA after 4 months. Efficacy was maintained over the 12-month trial period. After drug discontinuation, efficacy was maintained for about 2 months, and return to near pre-treatment levels occurred 4 to 6 months after discontinuation. In subjects with chemotherapy-induced hypotrichosis, 37.5% increase in efficacy was achieved at month 4 whereas only 18.2% of the vehicle treated subjects had a similar increase in GEA after 4 months. Efficacy was enhanced over the 12-month trial period.

In subjects with idiopathic hypotrichosis, 74.3% of the bimatoprost treated subjects had an increase in GEA of greater than 1 after 4 months, while only 13.6% of the vehicle treated subjects had a similar increase in GEA after 4 months. In subjects with chemotherapy-induced hypotrichosis, 72.9% of those receiving bimatoprost treatment had an increase of GEA of greater than 1 after 4 months, while 54.5% of the vehicle-treated subjects had a similar increase in GEA after 4 months (due to the natural untreated regrowth of eyelashes after cessation of chemotherapy). Both populations had statistically significant improvements in eyelash length, thickness/fullness, and darkness by bimatoprost compared with vehicle at months 4 and 6 (not shown).

The changes in eyelash length, thickness and darkness are shown in the table below.

### Eyelash length, thickness, and darkness Percent change from baseline at Month 4

| **Endpoint** | **Idiopathic Hypotrichosis (mean % change)** | | | **Chemotherapy-Induced Hypotrichosis (median % change) a** | | |
|---|---|---|---|---|---|---|
| | Bim 0.03% | Vehicle | P-value | Bim 0.03% | Vehicle | P-value |
| Length | 22.90% | -4.90% | <.001 | 28.50% | 11.30% | 0.022 |
| Thickness | 95.90% | -7.20% | <.001 | 180.10% | 25.00% | 0.002 |
| Darknessb | -15.70% | 1.40% | <.001 | -14.40% | -5.70% | 0.012 |

| | | | | | | |
|---|---|---|---|---|---|---|
| ^{a}Median values are provided because data from the post-chemotherapy subpopulation did not follow a normal distribution. ^{b}Negative change from baseline indicates darker lashes. | | | | | | |

### Results For Subjects With Idiopathic Hvopotrichosis

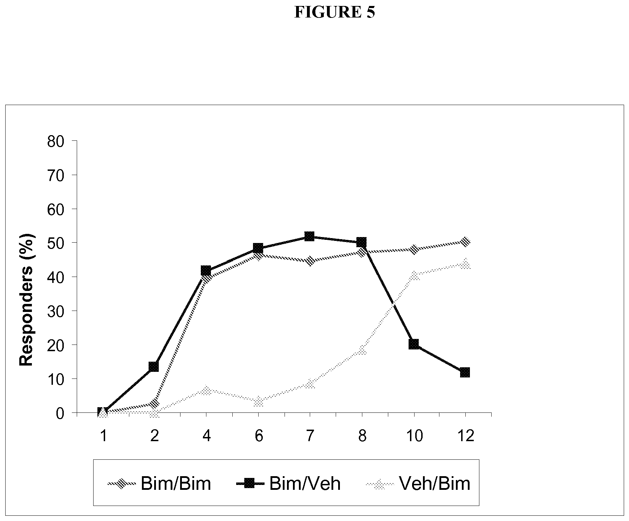

Fig. 5 is a plot of primary composite efficacy for the duration of the trial. Bim/Bim indicates subjects receiving bimatoprost for 12 months. Bim/Veh indicates subjects receiving bimatoprost for 6 months followed by vehicle for 6 months. Veh/Bim indicates subjects receiving vehicle for 6 months followed by bimatoprost for 6 months.

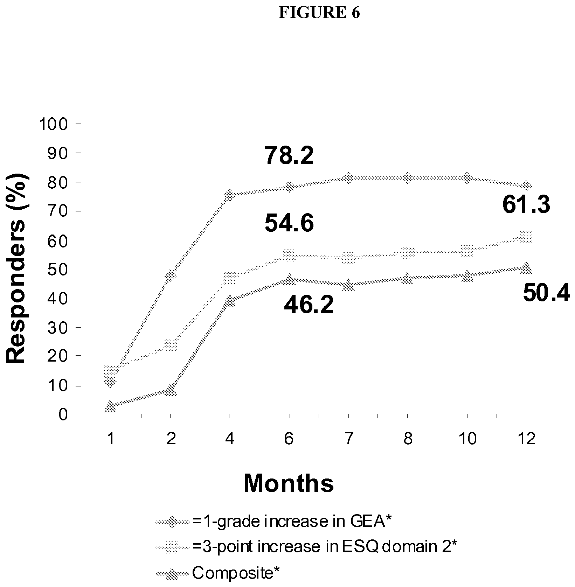

Fig. 6 is a plot of responder rates in bimatoprost-treated subjects by individual components of the primary composite efficacy measure for the duration of the trial. Figure 6 shows a GEA response rate of about 75% to about 80% and a maintenance of the effect and/or continuous improvement up to month 12.

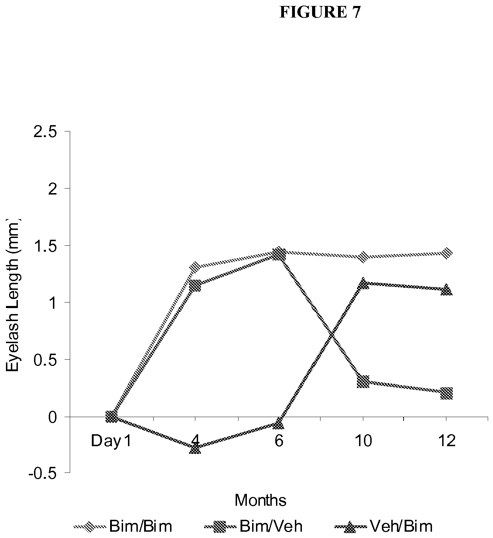

Fig. 7 is a plot of improvement in eyelash length for the duration of the trial. Bim/Bim indicates subjects receiving bimatoprost for 12 months. Bim/Veh indicates subjects receiving bimatoprost for 6 months followed by vehicle for 6 months. Veh/Bim indicates subjects receiving vehicle for 6 months followed by bimatoprost for 6 months.

### Results For Subjects With Chemotherapy-Induced Hvopotrichosis

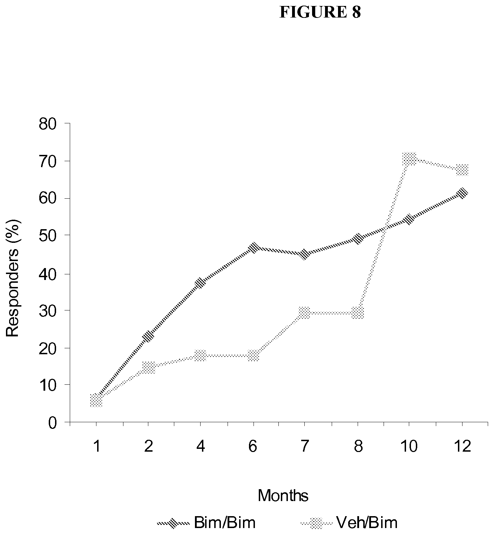

Fig. 8 is a plot of primary composite efficacy for the duration of the trial. Bim/Bim indicates subjects receiving bimatoprost for 12 months. Bim/Veh indicates subjects receiving bimatoprost for 6 months followed by vehicle for 6 months.

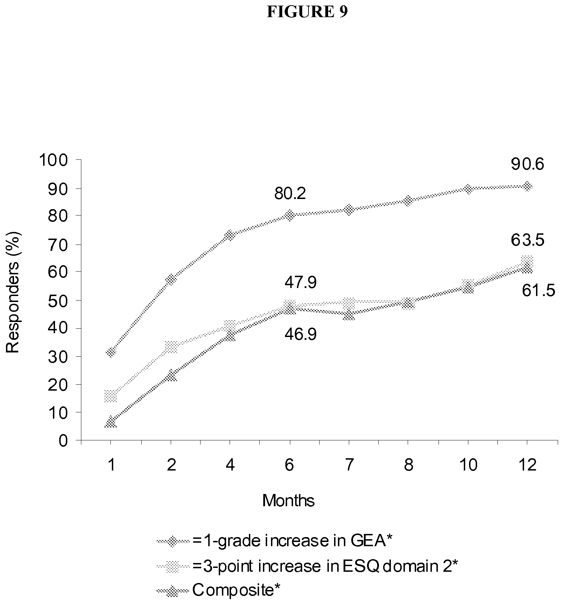

Fig. 9 is a plot of responder rates in bimatoprost-treated subjects by individual components of the primary composite efficacy measure for the duration of the trial. Figure 9 shows a GEA response rate of about 80% that is similar to the idiopathic population, and continuous improvement up to month 12.

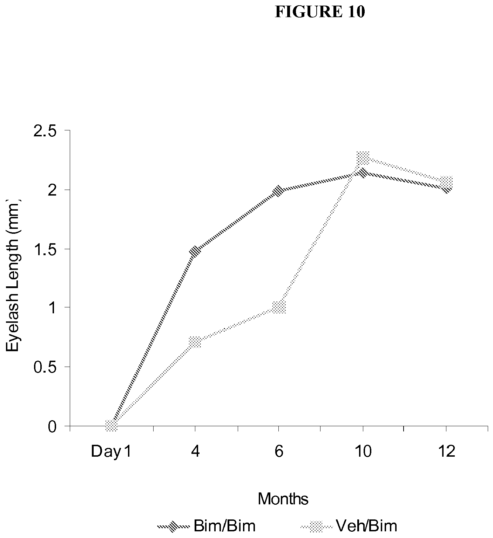

Fig. 10 is a plot of improvement in eyelash length for the duration of the trial.. Bim/Bim indicates subjects receiving bimatoprost for 12 months. Bim/Veh indicates subjects receiving bimatoprost for 6 months followed by vehicle for 6 months.

Over 12 months of bimatoprost treatment, the most common adverse events (>5%) in either idiopathic or post-chemotherapy population were conjunctival hyperaemia, punctate keratitis, eyelid pruritus, erythema of eyelids, and eye pruritus. Common adverse events (conjunctival hyperaemia, punctate keratitis, and eye pruritus) were reported at a higher rate in the post-chemotherapy population. This may have been related to the enduring effect of chemotherapy drugs on eyes. Common ocular and dermal adverse events occurred at a lower rate in the second 6-month trial period (months 6-12) compared with the first 6-month trial period. No drug-related serious adverse occurred in either subpopulation.

| 0 to 12 Months | | 0 to 6 Months | | 6 to 12 Months | | |
|---|---|---|---|---|---|---|
| Idiopathic Hypotrichosis | Post-Chemother apy | Idiopathic Hypotrichosis | Post-Chemotherapy | Idiopathic Hypotrichosis | Post-Chemotherapy | |
| (N=118) | (N=96) | (N=118) | (N=96) | (N=106) | (N=89) | |
| Eye disorders, n (%) | | | | | | |
| Conjunctival hyperaemia | 10 (8.5) | 16 (16.7) | 7 (5.9) | 15 (15.6) | 4 (3.8) | 1 (1.1) |
| Punctate keratitis | 3 (2.5) | 9 (9.4) | 3 (2.5) | 8 (8.3) | 0 (0.0) | 1 (1.1) |
| Eyelids pruritus | 7 (5.9) | 3 (3.1) | 6 (5.1) | 3 (3.1) | 1 (0.9) | 1 (1.1) |
| Erythema of eyelid | 6 (5.1) | 3 (3.1) | 2 (1.7) | 1 (1.0) | 5 (4.7) | 2 (2.2) |
| Eye pruritus | 1 (0.8) | 6 (6.3) | 1 (0.8) | 5 (5.2) | 0 (0.0) | 1 (1.1) |

| Skin and subcutaneous disorders, n (%) | | | | | | |
|---|---|---|---|---|---|---|
| Skin hyperpigmentation | 2 (1.7) | 5 (5.2) | 0 (0.0) | 3 (3.1) | 2 (1.9) | 2 (2.2) |

### Conclusions:

Bimatoprost ophthalmic solution 0.03% significantly increased eyelash growth in subjects with idiopathic as well as chemotherapy-induced hypotrichosis as measured by the primary composite endpoint (≥1-grade increase in the GEA score AND at least 3-point improvement in ESQ domain-2 score at week 16) and all secondary endpoints (eyelash length, thickness/fullness, and darkness). Bimatoprost treatment effects were maintained through the 12-month trial period. Bimatoprost treatment was safe and well-tolerated in the 2 populations. No new safety signals were detected in the 6- to 12-month trial period. Fewer common ocular and dermal AEs occurred in the second 6-month period than in the first 6 months of bimatoprost treatment. Efficacy was maintained for about 2 months after bimatoprost discontinuation; return to near pre-treatment levels occurred at about 4 to 6 months after discontinuation. Thus, daily application of bimatoprost ophthalmic solution to the eyelid margin over a one-year period was found to be safe, well tolerated and effective in both idiopathic and post-chemotherapy populations as assessed by several safety and efficacy measures.

## Claims

1. Bimatoprost for use in a method of growing eyelashes in chemotherapy patients, the method comprising applying 0.03% w/v bimatoprost to the eyelids before, during, or after chemotherapeutic treatment, wherein the method is applied for at least 6 months after completing chemotherapeutic treatment.

2. The bimatoprost for use according to claim 1 wherein the 0.03% bimatoprost is applied at least once a day.

3. The bimatoprost for use according to claims 1 and 2 wherein the method results in eyelashes which are longer, thicker or darker compared to patients receiving no treatment.

4. The bimatoprost for use according to claim 1 wherein the method is applied for at least 12 months after completing chemotherapeutic treatment.

5. The bimatoprost for use according to claim 3 wherein the number of eyelashes increases in comparison to post-chemotherapeutic patients who received no treatment.

6. The bimatoprost for use according to claim 1 wherein the bimatoprost is added during and after post-chemotherapeutic treatment.

7. The bimatoprost for use according to claim 2 wherein the method is applied twice a day.

8. The bimatoprost for use according to claim 7 wherein the bimatoprost is applied to the upper and lower eyelid margin of each eye.

9. The bimatoprost for use according to claim 1 wherein the method effectively treats post-chemotherapeutic hypotrichosis.

10. The bimatoprost for use according to claim 1 further comprising the step of administering one selected from the group consisting of Minoxidil® and Propecia®.

11. The bimatoprost for use according to claim 1 wherein the method results in lower incidence of conjunctival hyperemia, punctate keratitis, erythema of the eyelid, eye pruritis and skin hyperpigmentation than in patients receiving 0.03% w/v bimatoprost for treatment of idiopathic hypotrichosis.

12. Bimatoprost for use in a method of preventing loss of eyelashes in patients undergoing chemotherapy, the method comprising applying 0.03% w/v bimatoprost to the eyelids before, during, or after chemotherapeutic treatment, wherein the method is applied for at least 6 months after completing chemotherapeutic treatment.

13. The bimatoprost for use according to claim 12 wherein the 0.03% bimatoprost is applied at least once a day.

14. The bimatoprost for use according to claim 12 wherein the method results in eyelashes which are longer, thicker and darker compared to patients receiving no treatment.

15. The bimatoprost for use according to claim 12 wherein the method is applied for at least 12 months after completing chemotherapeutic treatment.

## Patentansprüche

1. Bimatoprost zur Verwendung bei einem Verfahren zum Wachsen von Wimpern bei Chemotherapiepatienten, wobei das Verfahren Applizieren von 0,03 % G/V Bimatoprost auf die Augenlieder vor, während oder nach chemotherapeutischer Behandlung umfasst, wobei das Verfahren während mindestens 6 Monate nach Abschluss einer chemotherapeutischen Behandlung appliziert wird.

2. Bimatoprost zur Verwendung gemäß Anspruch 1, wobei das 0,03-%ige Bimatoprost einmal täglich appliziert wird.

3. Bimatoprost zur Verwendung gemäß Anspruch 1 und 2, wobei das Verfahren zu dickeren, längeren oder dunkleren Wimpern führt im Vergleich zu Patienten, die keine Behandlung erhalten.

4. Bimatoprost zur Verwendung gemäß Anspruch 1, wobei das Verfahren während mindestens 12 Monate nach Abschluss einer chemotherapeutischen Behandlung appliziert wird.

5. Bimatoprost zur Verwendung gemäß Anspruch 3, wobei die Anzahl von Wimpern sich vergrößert im Vergleich zu postchemotherapeutischen Patienten, die keine Behandlung erhalten.

6. Bimatoprost zur Verwendung gemäß Anspruch 1, wobei das Bimatoprost während und nach post-chemotherapeutischer Behandlung zugegeben wird.

7. Bimatoprost zur Verwendung gemäß Anspruch 2, wobei das Verfahren zweimal täglich appliziert wird.

8. Bimatoprost zur Verwendung gemäß Anspruch 7, wobei das Bimatoprost auf den oberen und unteren Augenlidrand jedes Auges appliziert wird.

9. Bimatoprost zur Verwendung gemäß Anspruch 1, wobei das Verfahren post-chemotherapeutische Hypotrichose wirksam behandelt.

10. Bimatoprost zur Verwendung gemäß Anspruch 1, weiterhin umfassend den Schritt der Verabreichung von einem aus der Gruppe, bestehend aus Minoxidil® und Propecia®.

11. Bimatoprost zur Verwendung gemäß Anspruch 1, wobei das Verfahren zu niedrigerer Inzidenz von Bindehauthyperämie, punktförmiger Keratitis, Augenliderythem, Augenpruritis und Hauthyperpigmentierung führt, als bei Patienten, die 0,03 % G/V Bimatoprost zur Behandlung von idiopathischer Hypotrichose erhalten.

12. Bimatoprost zur Verwendung bei einem Verfahren zur Prävention von Wimpernverlust bei Patienten der Chemotherapie, wobei das Verfahren Applizieren von 0,03 % G/V auf die Augenlieder vor, während oder nach chemotherapeutischer Behandlung umfasst, wobei das Verfahren während mindestens 6 Monate nach Abschluss einer chemotherapeutischen Behandlung appliziert wird.

13. Bimatoprost zur Verwendung gemäß Anspruch 12, wobei das 0,03-%ige Bimatoprost mindestens einmal täglich appliziert wird.

14. Bimatoprost zur Verwendung gemäß Anspruch 12, wobei das Verfahren zu dickeren, längeren oder dunkleren Wimpern führt im Vergleich zu Patienten, die keine Behandlung erhalten.

15. Bimatoprost zur Verwendung gemäß Anspruch 12, wobei das Verfahren während mindestens 12 Monate nach Abschluss einer chemotherapeutischen Behandlung appliziert wird.

## Revendications

1. Bimatoprost pour utilisation dans un procédé de croissance de cils palpébraux chez des patients soumis à une chimiothérapie, le procédé comprenant l'application de 0,03 % en p/v de bimatoprost aux paupières avant, pendant ou après le traitement chimiothérapeutique, dans lequel le procédé est appliqué pendant au moins 6 mois après avoir effectué le traitement chimiothérapeutique.

2. Bimatoprost pour utilisation selon la revendication 1, dans lequel le bimatoprost à 0,03 % est appliqué au moins une fois par jour.

3. Bimatoprost pour utilisation selon les revendications 1 et 2, dans lequel le procédé permet d'obtenir des cils palpébraux qui sont plus longs, plus épais ou plus foncés en comparaison de patients ne recevant pas de traitement.

4. Bimatoprost pour utilisation selon la revendication 1, dans lequel le procédé est appliqué pendant au moins 12 mois après avoir effectué le traitement chimiothérapeutique.

5. Bimatoprost pour utilisation selon la revendication 3, dans lequel le nombre de cils palpébraux augmente en comparaison de patients post-chimiothérapeutiques qui n'ont pas reçu de traitement.

6. Bimatoprost pour utilisation selon la revendication 1, dans lequel le bimatoprost est ajouté pendant et après le traitement post-chimiothérapeutique.

7. Bimatoprost pour utilisation selon la revendication 2, dans lequel le procédé est appliqué deux fois par jour.

8. Bimatoprost pour utilisation selon la revendication 7, dans lequel le bimatoprost est appliqué à la marge des paupières supérieure et inférieure de chaque oeil.

9. Bimatoprost pour utilisation selon la revendication 1, dans lequel le procédé traite efficacement l'hypotrichose post-chimiothérapeutique.

10. Bimatoprost pour utilisation selon la revendication 1, comprenant en outre l'étape d'administration d'un produit choisi dans le groupe constitué du Minoxidil® et du Propecia®.

11. Bimatoprost pour utilisation selon la revendication 1, dans lequel le procédé a pour effet une moindre incidence de l'hyperémie conjonctivale, de la kératite ponctuée, de l'érythème de la paupière, du prurit oculaire et de l'hyperpigmentation de la peau que chez des patients recevant 0,03 % en p/v de bimatoprost pour le traitement de l'hypotrichose idiopathique.

12. Bimatoprost pour utilisation dans un procédé de prévention de perte de cils palpébraux chez des patients subissant une chimiothérapie, le procédé comprenant l'application de 0,03 % en p/v de bimatoprost aux paupières avant, pendant ou après le traitement chimiothérapeutique, dans lequel le procédé est appliqué pendant au moins 6 mois après avoir effectué le traitement chimiothérapeutique.

13. Bimatoprost pour utilisation selon la revendication 12, dans lequel le bimatoprost à 0,03 % est appliqué au moins une fois par jour.

14. Bimatoprost pour utilisation selon la revendication 12, dans lequel le procédé permet d'obtenir des cils palpébraux qui sont plus longs, plus épais et plus foncés en comparaison de patients ne recevant pas de traitement.

15. Bimatoprost pour utilisation selon la revendication 12, dans lequel le procédé est appliqué pendant au moins 12 mois après avoir effectué le traitement chimiothérapeutique.
